# FASCICULE DE BREVET EUROPEEN

(11) **EP 1 556 002 B1**
(45) Date de publication et mention de la délivrance du brevet: **22.10.2008**
(21) Numéro de dépôt: 03780245.1
(22) Date de dépôt: 29.10.2003
(51) Int. Cl.: A61K 9/00

(54) **STRUCTURE IMPLANTABLE POUR LA LIBERATION PROLONGEE ET CONTROLEE D'UN PRINCIPE ACTIF**
IMPLANTAT FÜR DIE VERZÖGERTE UND GESTEUERTE FREISETZUNG EINES WIRKSTOFFES
IMPLANTABLE STRUCTURE FOR PROLONGED AND CONTROLLED RELEASE OF AN ACTIVE PRINCIPLE

(30) Priorité: 30.10.2002 FR 0213623
(43) Date de publication de la demande: 27.07.2005
(73) Titulaire: Statice Sante, 25000 Besancon (FR); Pelissier, Edouard, 25870 Devecey (FR)
(72) Inventeur: PELISSIER, Edouard, F-25870 Devecey (FR)
(74) Mandataire: Hubert, Philippe
(86) Numéro de dépôt international: PCT/FR2003/003221
(87) Numéro de publication internationale: WO 2004/041241

(56) Documents cités:
- WO-A-00/24374
- WO-A-93/19739
- WO-A-93/20138
- WO-A-02/058670
- US-A- 5 922 340
- US-B1- 6 432 438

## Description

La présente invention concerne une structure implantable biorésorbable permettant la libération contrôlée d'un principe actif au niveau d'une lésion intracorporelle, notamment d'une incision chirurgicale, ainsi qu'un procédé de fabrication d'une telle structure.

L'invention trouve application dans le domaine médical et chirurgical.

Le traitement de la douleur, notamment en milieu hospitalier et en particulier après une intervention chirurgicale, est actuellement de plus en plus au coeur des préoccupations du personnel médical.

La douleur post-opératoire est due à la stimulation directe, par le traumatisme chirurgical, des terminaisons nerveuses libres, présentes dans tous les tissus et à la libération par les tissus traumatisés de substances algogènes, qui entretiennent la douleur par stimulation directe des terminaisons nerveuses et par abaissement du seuil d'activation des récepteurs nociceptifs.

Le traitement de la douleur post-opératoire par les antalgiques n'est qu'un traitement symptomatique, qui atténue la perception de la douleur au niveau des centres nerveux, sans agir à la source.

Les anesthésiques locaux ont une action directe au niveau des terminaisons nerveuses, ils interrompent totalement la transmission de la douleur par les fibres nerveuses. Leur efficacité est telle que l'on peut inciser et opérer au niveau d'une zone infiltrée par l'anesthésie locale.

Il a été démontré que l'infiltration de la plaie en fin d'intervention par un anesthésique local supprime efficacement la douleur post-opératoire, mais l'effet cesse avec la résorption du produit au bout de quelques heures (1-5).

D'autres études (6-10) ont confirmé l'intérêt de la méthode d'irrigation continue de la plaie par un anesthésique local au moyen d'un cathéter.

Cette méthode, si elle est efficace, présente toutefois l'inconvénient de comporter un appareillage encombrant qui limite l'activité et annule donc l'avantage de la réduction de douleur.

Le brevet US 6 432 438 décrit un système biodégradable comprenant au moins un polymère, qui peut être un copolymère d'acide lactique et d'acide glycolique et au moins un plastifiant. Ce système se présente sous forme semi-liquide ou visqueuse à laquelle un principe actif peut être incorporé juste avant l'utilisation. Un tel système qui n'est pas homogène ne peut qu'avoir un effet limité dans le temps.

Le document WO 00/24374 décrit une composition semi-liquide comprenant un copolymère thermoplastique, un solvant organique pharmaceutiquement acceptable, un principe actif et un additif pour contrôler la libération du principe actif comprenant nécessairement un PEG.

Le document WO 02/058670 concerne des formulations pour la libération contrôlée d'un anesthésique local, notamment la bupivacaïne. Ces formulations se présentent essentiellement sous forme de microsphères qui peuvent être administrées par implantation ou injection.

Le brevet US 6,063,405 concerne la préparation d'un système de délivrance à libération prolongée d'un principe actif, tel qu'un anesthésique, formé d'une matrice de polymère en suspension ou en solution dans l'eau, et destiné à être injecté.

On connaît par ailleurs d'après la demande de brevet WO 00 50004 une composition sous forme de gel ou d'une solution à faible viscosité à température ambiante, pour l'administration d'un anesthésique local lors d'une opération chirurgicale et notamment en chirurgie urologique.

Ce système et cette composition n'ont toutefois qu'un effet limité dans le temps.

L'invention vise à remédier à ces inconvénients, et à mettre à disposition une structure qui permette la libération progressive et contrôlée d'un principe actif au niveau d'une incision intracorporelle.

Une telle structure doit pouvoir être mise en place rapidement et facilement lors du temps de réparation, par exemple après une incision chirurgicale. Elle doit également se résorber le plus rapidement possible.

Ainsi, selon un premier aspect, l'invention concerne une structure implantable souple pour la libération prolongée et contrôlée d'un principe actif, constituée d'un support biorésorbable et d'un principe actif intimement lié audit support, et qui présente une cohésion entre le principe actif et le support biorésorbable induite par mouillabilité d'un des composants de la structure, et dans laquelle le support biorésorbable est formé d'un mélange de copolymère amorphe d'acide lactique et d'acide glycolique possédant un rapport massique entre les unités d'acide lactique et d'acide glycolique compris dans la gamme de 80/20 à 20/80, de préférence dans la gamme de 70/30 à 30/70, et de préférence encore égal à 50/50 et de 0,5 à 20 % en masse, de préférence de 5 à 15 % en masse par rapport à la masse du support, d'un plastifiant biocompatible choisi parmi l'acide lactique, un oligomère d'acide lactique, ou un mélange de ces composés, ledit mélange de copolymère et de plastifiant ayant une Tg inférieure ou égale à 15°C.

Le fondement de la présente invention réside donc dans le fait que la structure implantable possède une consistance souple et présente une cohésion (imbrication) entre le principe actif et le matériau du support biorésorbable induite par mouillabilité d'un des composants de la structure.

Conformément à l'invention, le support biorésorbable comprend à titre de composant principal un polyester aliphatique d'intérêt thérapeutique, c'est-à-dire qui est biocompatible, biorésorbable, bien toléré et qui n'entraîne pas d'effets adverses comme irritation locale, réaction allergique, réaction immunologique, toxicité systémique. Ces polyesters sont des copolymères d'acide lactique et d'acide glycolique (désignés sous la forme PLAₓGA_{y} ; x et y précisant respectivement les pourcentages des unités d'acides lactique et glycolique) à structure amorphe et de préférence à poids moléculaire peu élevé.

Avantageusement, on utilise un copolymère DL PLA-GA dans lequel le rapport massique entre les unités d'acides lactique et glycolique est compris dans la gamme de 80/20 à 20/80, de préférence 70/30 à 30/70. Un copolymère tout particulièrement préféré est celui comprenant des proportions égales en acides lactique et glycolique.

La structure implantable selon l'invention présente une bonne souplesse. Par « souplesse », on entend au sens de la présente invention que la Tg (température de transition vitreuse) du matériau support est inférieure ou égale à 15°C et peut de ce fait être manipulée aisément et sans présenter de rupture préjudiciable lors de la mise en place dans la plaie opératoire.

Cette souplesse est une caractéristique des matériaux polymères à propriétés dites viscoélastiques. Elle traduit, pour un même matériau porté à des températures T la transition en fonction de T entre des structures dites "rigides - élastiques" pour des températures inférieures à la température de transition vitreuse (T < Tg) et des structures dites "élastiques - caoutchoutiques" pour des températures supérieures à la température de transition vitreuse (T > Tg). Cette propriété est couramment étudiée à l'aide de viscoélasticimètres où la transition citée est définie par la variation caractéristique, en fonction de la température, d'un module élastique de type module d'Young ou module de cisaillement.

Lorsque le polyester aliphatique d'intérêt thérapeutique est un copolymère d'acide lactique et d'acide glycolique, pour lequel la Tg est proche de la température ambiante, la souplesse du matériau support est ajustée de manière bien connue de l'homme du métier par l'adjonction d'un plastifiant biocompatible. Selon l'invention, ce plastifiant est choisi parmi l'acide lactique, les oligomères d'acide lactique notés couramment (OLA), et les mélanges de ces composés. Le plastifiant est généralement ajouté en une quantité comprise dans la gamme de 0,5 % à 20 % en masse, de préférence de 5 à 15 % en masse, par rapport à la masse du support.

Le principe actif qui peut être utilisé dans le cadre de l'invention n'est pas limité à un principe actif particulier, et peut être avantageusement choisi parmi les anesthésiques locaux, les antalgiques morphiniques ou non morphiniques, les facteurs de cicatrisation, les anti-inflammatoires, les antibiotiques, les anti-fongiques, les corticoïdes, les hormones, les antimitotiques, les facteurs de croissance. Un mélange de principes actifs est de plus possible afin de :
- moduler l'efficacité temporelle des principes actifs,
- assurer différentes fonctions thérapeutiques cumulatives.

On peut ainsi concevoir l'apport d'anti-inflammatoires ou de corticoïdes au niveau ou au voisinage d'une articulation, de substances antiseptiques et/ou cicatrisantes au niveau d'une plaie chronique, d'antimitotiques au niveau d'une tumeur inextirpable ou de métastases, ou de substances stimulantes au niveau d'une structure nerveuse.

De manière particulièrement préférée, le principe actif est un anesthésique local comme par exemple la lidocaïne, la bupivacaïne, la benzoïne, la tétracaïne, la mepivacaïne, la ropivacaïne. On peut ajouter à l'anesthésique local une substance capable de prolonger son action, comme la clonidine ou le fentanyl.

La quantité de principe(s) actif(s) ne dépasse généralement pas 60 % de la masse du support et varie en fonction de la nature même du principe actif et du but thérapeutique recherché.

La structure biodégradable conforme à l'invention peut être implantée au niveau des plans de suture de tissus incisés, et permet un apport *in situ* de principe actif à une concentration définie, pendant une durée spécifique au but thérapeutique recherché.

Cette structure résiste à la stérilisation et au stockage sous différentes conditions climatiques.

Dans le cas où le principe actif est un anesthésique local, la structure implantable selon l'invention permet de supprimer ou réduire au minimum la douleur, et tout particulièrement la douleur post-opératoire, avec tous les avantages qui en découlent : confort idéal pour le patient, réduction au minimum des soins post-opératoires, réduction du risque thrombo-embolique par la reprise précoce de la marche, raccourcissement de la durée d'hospitalisation et donc des coûts. Elle favorise également la pratique de la chirurgie ambulatoire et une reprise d'activité rapide du patient.

La structure implantable selon l'invention est fabriquée au moyen d'un procédé thermo-mécanique de mise en forme, qui permet d'obtenir une cohésion (imbrication) entre le principe actif et le matériau du support biorésorbable induite par mouillabilité d'un des composants de la structure.

Lorsque le principe actif est soumis à un passage par une phase liquide, qui induit cette mouillabilité, lors de l'élévation de température au dessus de son point de fusion imposé par une des étapes du procédé de fabrication, la cohésion recherchée est implicite.

Lorsque le principe actif conserve sa forme solide tout au long du procédé de fabrication, c'est le matériau du support qui doit être soumis à un passage par une phase liquide voire visqueuse lors du procédé de fabrication.

Le principe actif et le matériau du support peuvent tous deux subir concomitamment ce changement de phases si la température de fusion du principe actif et la température de transition vitreuse ou de fusion du matériau support, sont voisines.

Ledit changement de phases, pour obtenir l'imbrication recherchée, est généralement réalisé dans une chambre dite "chambre de transfert".

Ainsi, selon un second aspect, l'invention concerne un procédé de fabrication d'une structure implantable souple pour la libération prolongée et contrôlée d'un principe actif, constituée d'une structure composite homogène avec des interfaces cohérentes, et dans laquelle le support biorésorbable est formé d'un mélange de copolymère amorphe d'acide lactique et d'acide glycolique possédant un rapport massique entre les unités d'acide lactique et d'acide glycolique compris dans la gamme de 80/20 à 20/80, de préférence dans la gamme de 70/30 à 30/70, et de préférence encore égal à 50/50 et de 0,5 à 20 % en masse, de préférence de 5 à 15 % en masse, par rapport à la masse du support, d'un plastifiant biocompatible choisi parmi l'acide lactique, un oligomère d'acide lactique, ou un mélange de ces composés, ledit mélange de copolymère et de plastifiant ayant une Tg inférieure ou égale à 15°C, qui comprend les étapes suivantes :
a) un mélange des produits composant la structure,
b) un passage avec ou sans pression appliquée, dans une chambre de transfert, soit b1) à une température comprise entre la température de fusion du principe actif et la température de transition vitreuse ou de fusion du copolymère, soit b2) à température supérieure à la fois à la température de fusion du principe actif et à la température de transition vitreuse du copolymère, et
c) une mise en forme de la structure implantable sous pression à partir de cet état intermédiaire.

Ces différentes étapes peuvent être complétées si nécessaire par un traitement thermique final.

A l'étape a), les produits initialement à l'état solide sont avantageusement soumis au préalable à un traitement de dessiccation.

Le changement de phases est réalisé à l'étape b). Ce changement de phases est de type :
■ solide - liquide dans le cas où il est induit préférentiellement par le principe actif seul ou le matériau support seul,
■ solide - visqueux ou solide - visqueux - liquide dans le cas où il est induit préférentiellement par un matériau polyester aliphatique amorphe ou, respectivement, semi-cristallin,
■ liquide - liquide lorsqu'il est induit par la fusion concomitante du principe actif et du matériau support.

A l'issue du procédé, les produits mis en forme sont démoulés, de préférence sur sole réfrigérée.

On comprend donc que le procédé de l'invention ne fait pas intervenir de solvant dans les différentes étapes indiquées ci-dessus.

Lorsque la différence entre la température de fusion (Tf) du principe actif et la température de transition vitreuse ou de fusion du polyester aliphatique (suivant sa nature amorphe ou semi-cristalline) est importante, le changement de phases de l'étape b) s'opère avantageusement à une température comprise entre la Tf du principe actif et la Tg ou Tf du polyester aliphatique, de préférence à une température proche de la Tf du principe actif.

Lorsque la différence entre la Tf du principe actif et la Tg ou la Tf du polyester aliphatique est faible (environ de l'ordre de 10 à 15°C au plus), le changement de phases de l'étape b) s'opère avantageusement à une température supérieure à la fois à la Tf du principe actif et à la Tf ou la Tg du polyester aliphatique.

Le procédé conforme à l'invention permet d'obtenir une structure composite homogène avec des interfaces cohérentes (c'est-à-dire sans décohésion interfaciale), qui présente de plus un point de fusion ou de transition vitreux bas (inférieur à celui du polyester aliphatique).

A titre d'exemple de procédé conforme à l'invention, on peut citer ceux décrits notamment dans « La mise en forme des matières plastiques : J.F. AGASSANT, P. AVENAS, J.Ph. SERGENT, Ed. Lavoisier, 1989 » ou dans « Matières plastiques : J.P. TROTIGNON, J. VERDU, A. DOBRACZYNSKI, M. PIPERAUD, Ed. Nathan 1996 », en particulier les procédés de type :
- moulage par compression-transfert,
- moulage par injection-transfert,
- extrusion ou filage avec étape préliminaire de transfert.

De préférence, on utilise le procédé du type moulage par compression - transfert, qui est un procédé consistant à introduire sous pression un matériau dans un état de fluidité donné à l'intérieur de la cavité d'un moule et qui se décompose classiquement en quatre étapes :
- la plastification : la matière, introduite préalablement dans un creuset, est amenée partiellement ou totalement par chauffage à un état fluide et homogène (changement de phases),
- l'injection : un piston permet l'introduction de cette matière fluidisée dans le moule,
- la conformation : la matière peut alors être conformée dans le moule avec une cinétique de durcissement rapide,
- le démoulage final.

Avantageusement, la granulométrie du mélange des produits de départ est contrôlée dans la gamme comprise entre 5 et 150 µm, de préférence entre 10 et 50 µm. En règle générale, l'utilisation de broyeurs mécaniques ou de broyeurs à jet d'air est conseillée. De plus, il est recommandé de travailler dans des domaines de températures de mise en forme où le principe actif conserve son intégrité structurale et ses propriétés thérapeutiques.

Le moulage par injection - transfert procède de la même démarche expérimentale. L'extrusion - transfert est par ailleurs une technologie connue.

La morphologie des structures implantables peut être variée et conduire à la fabrication de fils, de films, de joncs, de rubans (notamment de forme parallélépipédique base carrée ou rectangle), de mèches, de tissus tissés ou non-tissés, de plaques, de cathéters ou de comprimés voire de tablettes. On peut concevoir une autre forme, par exemple un film qui pourrait être appliqué dans les chirurgies mammaires ou la chirurgie anale ainsi que le traitement des brûlures et autres lésions cutanées. On peut également envisager une structure où le(s) principe(s) actif(s) serai(en)t incorporé(s) dans un fil de suture.

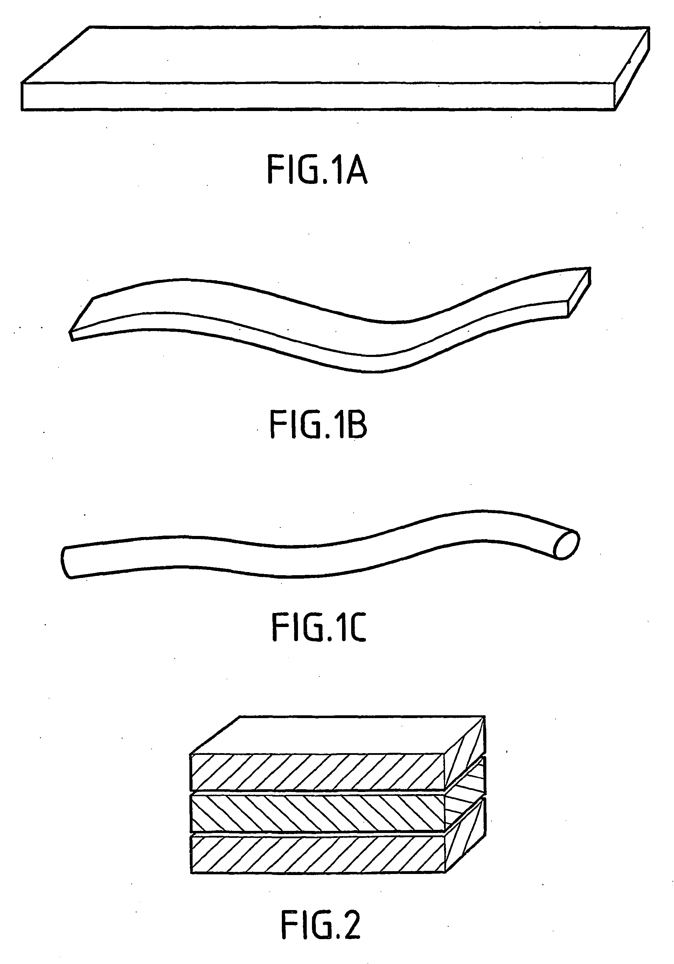

Différentes structures selon l'invention sont représentées sur les figures 1A (ruban), 1B (ruban ondulé) et 1C (jonc).

De plus, des structures composites mixtes comprenant la structure implantable selon l'invention, peuvent être élaborées dans le but d'obtenir plusieurs cinétiques de libération complémentaires, par exemple une cinétique de libération importante dans les premières heures qui suivent l'acte chirurgical, suivie d'une cinétique de libération retardée ensuite.

La morphologie spécifique en résultant est classiquement effectuée via la réalisation de structures sandwich telle que celle représentée sur la figure 2.

Avantageusement, la surface d'échange du produit biorésorbable avec le liquide interstitiel peut être augmentée pour accélérer sa biodégradabilité et favoriser ainsi la perméation aux fluides et au principe actif, en réalisant par exemple des structures à forme géométrique définie (cylindre, parallélépipède) avec une topographie superficielle présentant des ondulations ou des rugosités de surface formées de dessins géométriques répétitifs ou aléatoires dans le but d'augmenter la surface spécifique des structures implantables.

Le site d'insertion de la structure implantable est fonction du type de lésion ou d'incision :

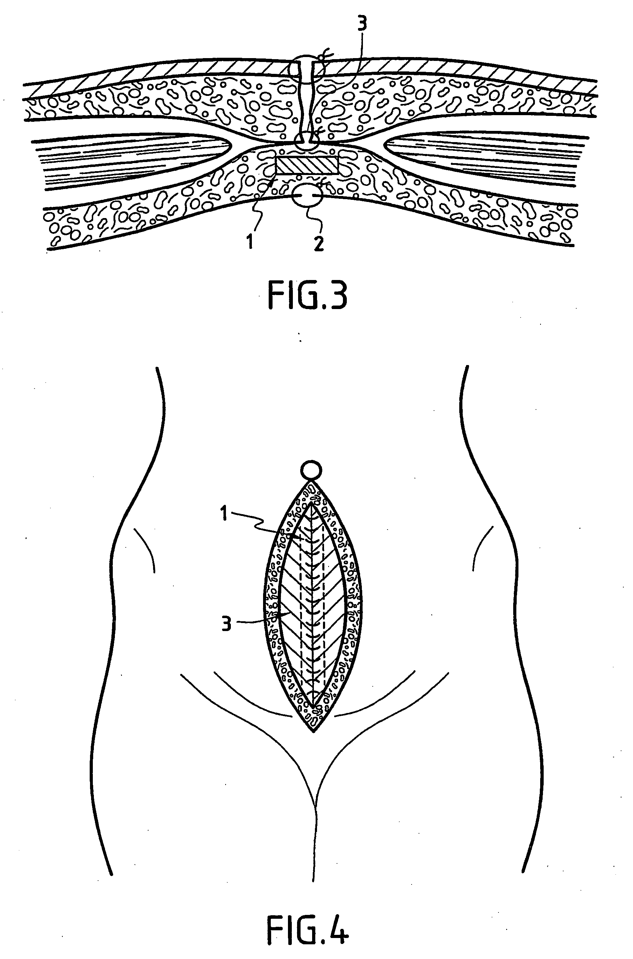

Les figures 3 et 4 représentent schématiquement une laparotomie médiane : la structure implantable (1) est placée longitudinalement, dans le sens de l'incision, entre le plan de suture du péritoine (2) et celui de l'aponévrose (3), dans l'espace pré-péritonéal.

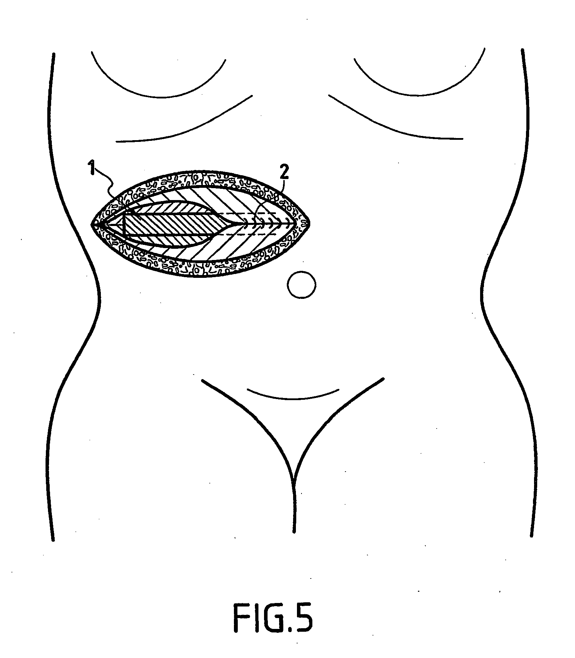

La figure 5 représente schématiquement une laparotomie transversale : la structure implantable (1) est placée dans la loge musculaire, à la face profonde du plan de suture aponévrotique (2).

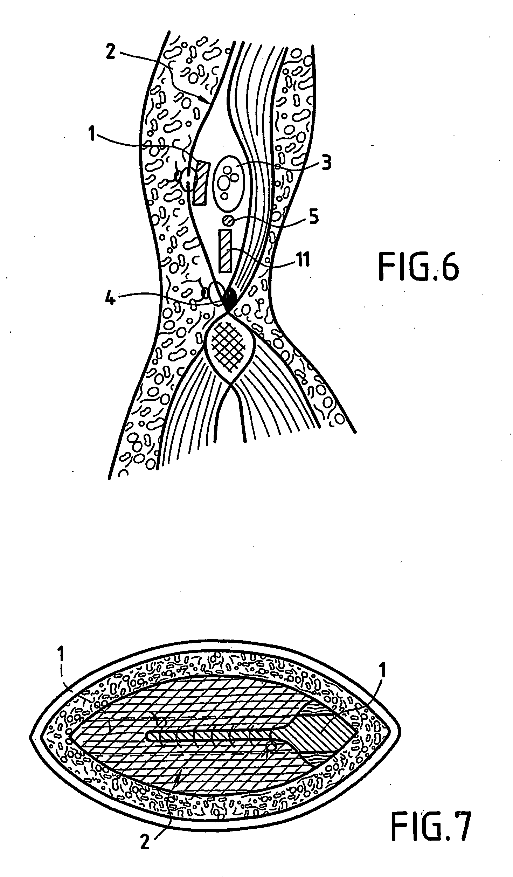

La figure 6 représente schématiquement une herniorraphie : un morceau de l'implant (1) est placé à la face profonde de l'aponévrose du grand oblique (2) et un autre (11) au bord inférieur du cordon spermatique (3), au contact du plan de suture profond (4) et du rameau génital du nerf génito-crural (5). La figure 7 est une coupe antéro-postérieure de la représentation de la figure 6.

Pour d'autres types d'incisions, au niveau des membres ou du thorax, l'implant sera généralement placé au contact des sutures musculaires ou aponévrotiques.

Pour les strippings de varices, il peut être attiré par le stripper dans le trajet du stripping où il sera laissé en place.

D'autres formes adaptées à différents types de chirurgie peuvent être conçues ultérieurement, notamment sous forme de film pour de grands décollements cellulaires.

L'homme de métier comprendra aisément à la lecture de la présente description que les dimensions de la structure implantable ainsi que la quantité de principe actif à incorporer à cette structure dépendront de la nature de l'application envisagée.

La quantité de principe actif libéré dépend de la masse de support biorésorbable et de la concentration initiale de ce principe actif dans le support.

A titre indicatif, pour le traitement de la douleur post-opératoire, la quantité d'anesthésique local de type lidocaïne est d'environ 3 g dans le but de libérer d'une manière continue au maximum environ 600 mg par vingt quatre heures de ce produit sur cinq jours.

Cette quantité peut être diminuée en fonction de la plus grande activité reconnue de certains anesthésiques locaux, comme la bupivacaïne, la mepivacaïne, la ropivacaïne.

A titre indicatif, les implants conformes à l'invention ont généralement une longueur comprise entre 3 et 25 cm et de préférence voisine de 7 cm, grandeur qui convient pour beaucoup d'incisions courantes. Si, pour des interventions plus importantes, nécessitant des incisions de 15 à 20 cm, on souhaite libérer la même dose quotidienne d'anesthésique local pendant la même durée et que la composition de l'implant est la même, il faut évidemment réduire ses deux autres dimensions :
■ pour un implant de 14 cm de longueur et 0,22 cm d'épaisseur, à masse et à volume égaux, la largeur doit être de 0,75 cm. On peut également utiliser deux implants de 7 x 0,22 x 0,75 cm.
■ pour un implant de 21 cm de longueur et 0,22 cm d'épaisseur, la largeur doit être de 0,5 cm. On peut également utiliser trois implants de 7 x 0,22 x 0,5 cm.

On peut également augmenter la longueur de l'implant, ce qui offre la possibilité d'un volume plus grand pour une épaisseur et une largeur du même ordre que dans l'exemple de base, pour libérer plus de produit actif. Dans ce cas, on modifie bien évidemment la composition de l'implant en conséquence.

L'invention sera décrite plus en détail à l'aide des exemples ci-après, donnés à titre purement illustratif.

### EXEMPLE 1 : Influence de l'ajout d'un plastifiant sur la Tg

Le Tableau 1 ci-après regroupe les températures de transition vitreuse (Tg) et de fusion (Tf) de deux polymères utilisables comme support biorésorbable dans le cadre de l'invention de type PLA₅₀GA₅₀, matériau amorphe intrinsèquement biodégradable.

Les différences de valeurs de Tg relevées sur les PLA₅₀GA₅₀ sont liées à leur composition nominale et en particulier à leur masse moléculaire (75000 g/mol pour le copolymère commercialisé par la Société PURAC, 65000 g/mol pour le copolymère commercialisé par la Société MEDISORB).

En règle générale pour chaque lot de PLAGA, la valeur de la Tg est systématiquement évaluée.

**Tableau 1**

| **Matériau** | **Tg (°C)** | **Tf (°C)** |
|---|---|---|
| **PLA₅₀GA₅₀ (MEDISORB)** | 31 | |
| **PLA₅₀GA₅₀ (PURAC)** | 45 | |

Le Tableau 2 donne les valeurs de Tg (°C) des copolymères PLA₅₀GA₅₀ additionnés de 5, 10 ou 15% en masse d'acide lactique.

**Tableau 2**

| **% d'acide** | **PLA₅₀GA₅₀** | **PLA₅₀GA₅₀** |
|---|---|---|
| **lactique** | **(MEDISORB)** | **(PURAC)** |
| **0** | 31 | 45 |
| **5** | 20 | 41 |
| **10** | 15 | 20 |
| **15** | 3 | 16 |

Les résultats des Tableaux 1 et 2 montrent qu'il est possible de contrôler la « souplesse » du PLA₅₀GA₅₀ à température ambiante par addition d'un plastifiant.

### EXEMPLE 2 : Optimisation des conditions de mise en forme de structures biorésorbables

Le but de cet exemple est la fabrication d'un ruban de forme parallélépipédique permettant de libérer théoriquement d'une manière contrôlée une quantité de principe actif de 500 mg/j pendant 3 jours soit au total 1500 mg.

Il a été vérifié en préalable qu'il était possible d'incorporer dans la structure implantable, quel que soit le procédé de mise en forme retenu, environ 50% de principe actif dans 50% du matériau de support en conservant la souplesse recherchée.

Le poids total de l'implant est, dans ces conditions, de 3 g. Pour une densité moyenne de l'implant estimée à 1,3, le volume de l'implant sera de 2,31 cm³. Ce volume correspond par exemple à un parallélépipède de dimension :
longueur imposée : 7 cm
largeur imposée : 1,5 cm
épaisseur : 0,22 cm (2,2 mm)

La granulométrie des anesthésiques locaux présentant une dispersion en taille généralement comprise dans l'intervalle 10-500 µm, il est nécessaire de procéder au préalable au broyage de ces produits pulvérulents suivi d'un passage à l'étuve (une heure à 40°C par exemple), pour obtenir une granulométrie finale dans l'intervalle 10-50 µm.

Les conditions opératoires générales suivantes ont été retenues pour le copolymère PLA₅₀GA₅₀ pour obtenir cette morphologie particulière par moulage par compression - transfert :
■ température de plastification : 80°C-90°C,
■ pression d'injection dans le moule : 60 bars-100 bars,
refroidissement final sur plaque réfrigérante revêtue de téflon avant démoulage.

### EXEMPLE 3 : Cinétiques in vitro de libération de lidocaïne à partir d'un implant à base de PLA₅₀GA₅₀

Ces cinétiques ont été réalisées sur une masse de 3 g de ruban préparé selon les conditions opératoires de l'exemple 2A/, pouvant libérer une quantité de 1500 mg de lidocaïne (fabrication par moulage par compression - transfert). Ce ruban est immergé sous agitation magnétique dans 500 ml de tampon PBS à pH=7,4, à la température de 37°C, le milieu d'immersion étant changé toutes les 24 h pour éviter une saturation de ce dernier en produit libéré.

Des compositions contenant respectivement en pourcentage en masse :
85% de PLA₅₀GA₅₀ et 15% d'acide lactique (85/15) ou
90% de PLA₅₀GA₅₀ et 10% d'acide lactique (90/10) ou
95% de PLA₅₀GA₅₀ et 5% d'acide lactique (95/5)
ont été testées.

Les valeurs des pentes des courbes de libération sont regroupées dans le tableau 4.

La méthode de dosage in vitro de l'anesthésique libéré de la structure implantable est la spectrométrie UV (PERKIN ELMER Lambda 20). La raie caractéristique est située à la longueur d'onde égale à 263 nm. Le dosage est réalisé en mode continu ou discontinu en changeant le bain d'immersion toutes les 24 h.

**Tableau 4**

| **Composition** | **mg / 24 h** | **mg / 48 h** | **mg / 72 h** | **mg / 96 h** |
|---|---|---|---|---|
| **PLA₅₀GA₅₀ / acide lactique** | | | | |
| **85/15** | 600 | 900 | 1200 | 1500 |
| **90/10** | 500 | 900 | 1200 | 1500 |
| **95/5** | 450 | 800 | 1100 | 1400 |

Un traitement thermique préalable de l'implant à 40°C pendant 2 heures montre un comportement plus homogène de la cinétique de libération par période de 24 h de l'ordre de 350 mg/24 h.

### EXEMPLE 4 (pour référence): Cinétique in vitro de libération de Bupivacaïne à partir d'un implant à base de PLA₅₀GA₅₀

A partir d'un lot de quatre implants identiques de forme parallélépipédique base carrée de dimention 70 x 5 x 5 mm, de masse égale à 2,7 g contenant 50 % de Bupivacaïne; il a été procédé à des tests complémentaires de libération de cet anesthésique local. Ces tests ont été conduits par immersion de l'implant dans 500 cm³ de tampon PBS (pH = 7,4) à la température de 37°C cette fois-ci en l'absence d'agitation magnétique. L'agitation magnétique n'étant réalisée qu'au moment des dosages pour uniformiser le milieu liquide avant prélèvement d'une quote-part de celui-ci pour analyse.

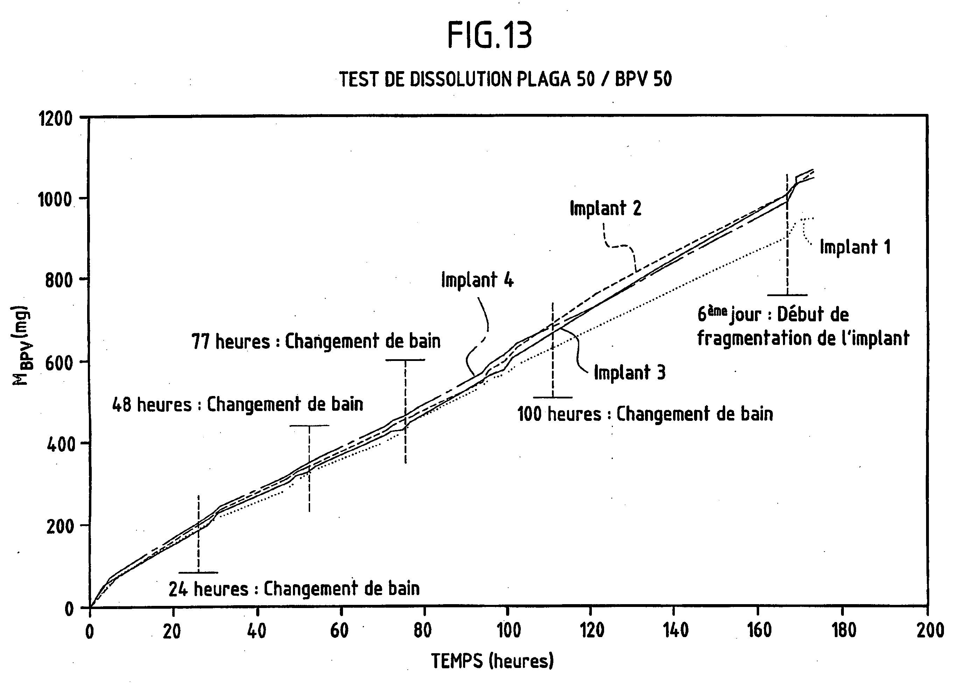

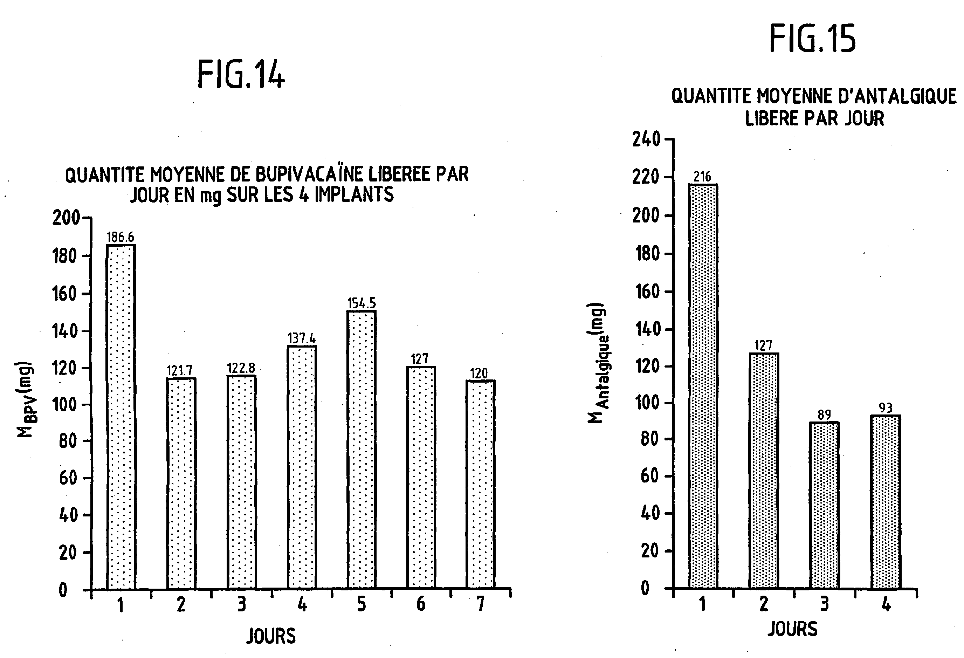

La courbe de libération sur une période de 6 jours est montrée sur la figure 13 avec en complément un graphe précisant la quantité libérée par période de 24 heures (cf. figure 14).

Comme attendu, la libération est plus importante le premier jour et reste sensiblement de valeur constante les autres jours de l'essai.

Il a été constaté en complément qu'après une période d'immersion de 6 jours, les implants étaient complètement destructurés.

### EXEMPLE 5 (pour référence): Cinétique in vitro de libération d'un mélange d'anesthésiques locaux à partir d'un implant à base de PLA₅₀GA₅₀

La même expérimentation que celle décrite dans l'exemple 4 a été conduite à partir d'un implant constitué au niveau de la concentration en anesthésiques locaux de :
■ 5 % de Lidocaïne,
■ 45 % de Bupivacaïne,
■ 50 % de PLA₅₀GA₅₀.

Le graphe précisant la quantité libérée par période de 24 heures est montré figure 15. Il est noté une cinétique de libération moins conséquente que celle constatée dans l'exemple 4.

### EXEMPLE 6 : Cinétique in vitro de libération d'anesthésiques locaux à partir d'un implant à base de PLA₅₀GA₅₀ de structure "sandwich"

A l'aide de la technique de moulage-transfert il a été procédé à la réalisation d'un implant de structure "sandwich" identique à celle présentée figure 2. La structure périphérique (l'enveloppe) est formée de deux rubans de 1,5 g présentant comme matériau support un mélange 95/5 (massique) de PLA₅₀GA₅₀ et d'acide lactique et contenant en proportions relatives 25% en masse de lidocaïne. La structure interne (l'âme) est élaborée avec le même matériau support mais contient en proportions relatives 50% en masse de bupivacaïne.

Pour chaque structure de forme parallélépipédique (longueur 7 cm, largeur 1 cm, épaisseur 1 mm) le temps dans la chambre de transfert portée à 85°C est de 15 min. La pression de moulage sur plaque revêtue de téflon est de 65 bars. Le tableau 9 précise les données obtenues.

**Tableau 9**

| **mg/24 h** | **mg/48 h** | **mg/72 h** |
|---|---|---|
| 550 | 490 | 510 |

Une cinétique de libération quasi linéaire est constatée.

### EXEMPLE 7 : Cinétiques in vitro de déstructuration d'un implant constitué de PLA₅₀GA₅₀ / acide lactique / bupivacaïne

Le tableau 10 précise en final les cinétiques de déstructuration, dans du milieu PBS (pH=7,4) porté à 37°C, d'un implant élaboré par les techniques de moulage par compression - transfert de copolymère PLA₅₀GA₅₀ / acide lactique / bupivacaïne (BPV). La quantité de bupivacaïne a été conservée constante et égale à 1,5 g pour un implant de masse totale égale à 3 g.

**Tableau 10**

| | **2 jours** | **4 jours** | **15 jours** |
|---|---|---|---|
| **85 / 15 / BPV** | matériau devenant de consistance molle | matériau diminuant de volume | solubilisation totale |
| **90 / 10 / BPV** | " | " | " |
| **95 / 5 / BPV** | " | " | solubilisation dès 10 jours d'immersion |

Les mécanismes associés à cette cinétique de déstructuration ont été appréhendés via l'observation par microscopie électronique à balayage de coupes de rubans réalisées par cryofracture.

Le microscope utilisé est un microscope électronique à balayage à émission de champ (JEOL - 6400F) équipé d'une platine pour préparation de l'échantillon par cryofracture (OXFORD CT 1500 HF). Le protocole opératoire est le suivant :
■ dans une chambre de congélation indépendante du microscope l'échantillon est immergé dans l'azote liquide et mis sous vide primaire (10⁻³ torr),
■ cet échantillon est ensuite transféré dans une chambre où règne un vide secondaire (10⁻⁶ torr),
■ il subit une fracture à l'aide d'un système contondant,
■ une sublimation de la glace pouvant se trouver sur l'échantillon est réalisée,
■ un dépôt d'or (100 Å) est effectué sur l'échantillon fracturé,
■ le transfert sur la platine du microscope est réalisé en final sans remise à l'air.

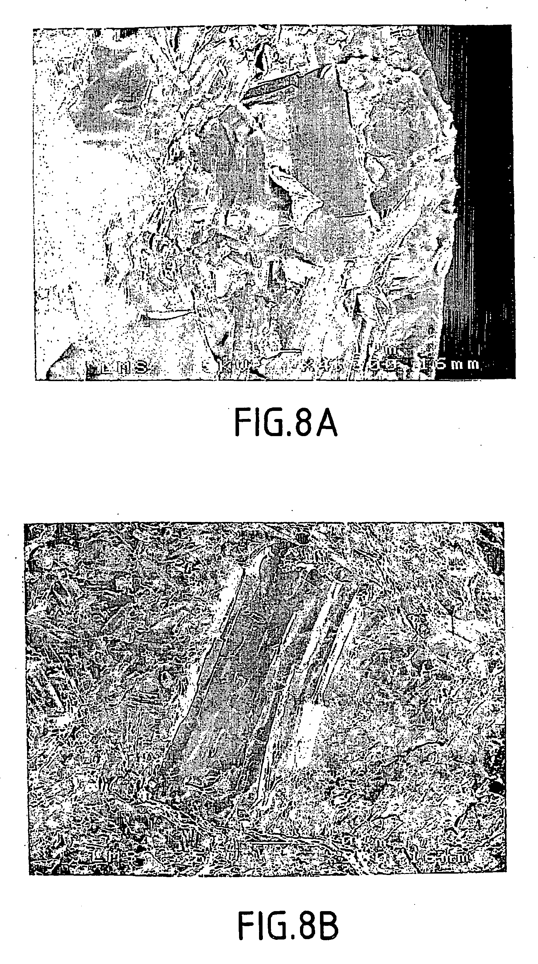

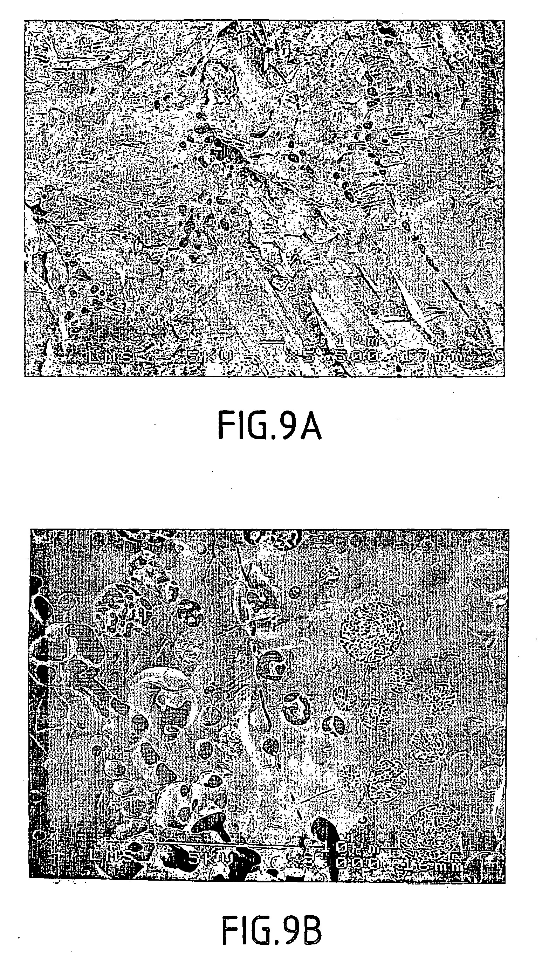

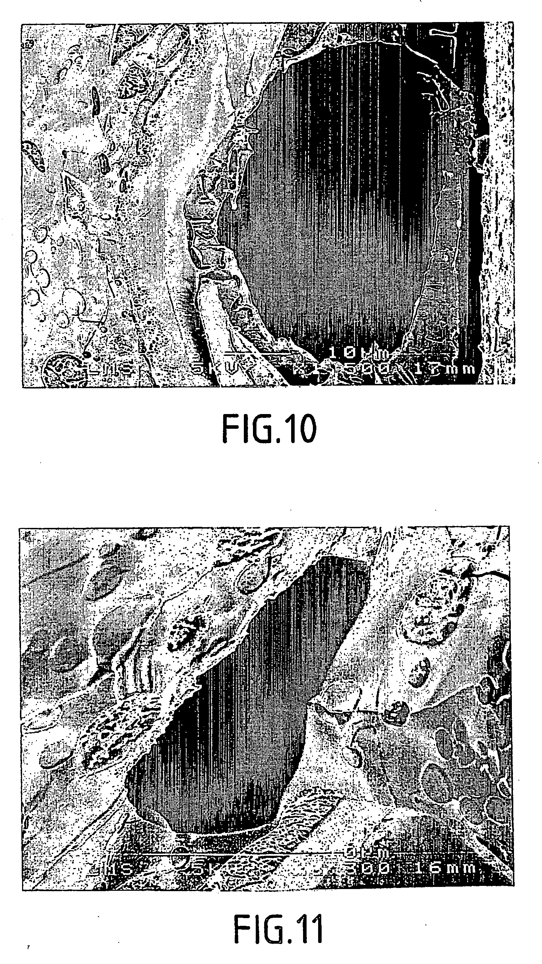

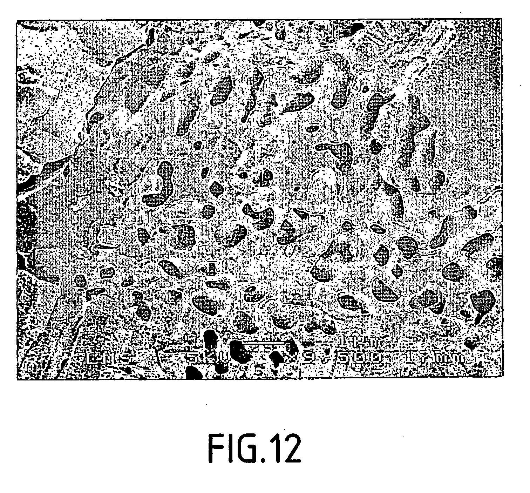

Les données expérimentales suivantes ont ainsi été obtenues :
■ en préalable, il a été vérifié que la densité du principe actif solide, (bupivacaïne dans l'exemple illustré) était homogène en surface et à coeur de l'échantillon, et que les interfaces entre le principe actif et le support polymère apparaissaient parfaitement cohérentes (figures 8A et 8B),
■ après un temps d'immersion de quelques heures (2 à 5 heures) dans le milieu PBS (pH = 7,4, T = 37°C), la présence de microporosités locales associables à la dissolution totale du principe actif positionné à la surface de l'implant est visualisée (figures 9A et 9B),
■ après un temps d'immersion de 9 heures, il est relevé une dissolution totale du principe actif positionné à la surface de l'implant (figure 10),
■ après un temps d'immersion de 12 heures la dissolution du principe actif se poursuit pour atteindre progressivement le coeur du matériau (figure 11),
■ concomitamment, le nombre de microporosités et de nanoporosités dans le polymère s'accroît (figure 12).

Il apparaît ainsi que c'est la déstructuration locale du polymère par formation de porosités ouvertes qui favorise la solubilisation du principe actif et donc sa libération temporelle sous forme dissoute.

### REFERENCES BIBLIOGRAPHIQUES

**1.** Nehra et al.. Pain relief after inguinal hernia repair : a randomized double-blind study. Br J Surg 1995 ; 82 : 1275-7.
**2.** Ding et al.. Post-herniorraphy pain in outpatients after pre-incision ilioinguinal-hypogastric nerve block during monitored anaesthesia care. Can J Anaesth 1995 ; 42 : 12-15.
**3.** Reid et al. Day-case hemiotomy in children. A comparison of ilio-inguinal nerve block and wound infiltration for postoperative analgesia. Anaesthesia 1987; 42 : 658-61.
**4.** Lafferty et al. A comparison of postoperative pain relief techniques in orchidopexy. Ann R Coll Surg Engl 1990; 72 : 7-8.
**5.** Wright. Controlled trial of wound infiltration with Bupivacaine for postoperative pain relief after appendicectomy in children. Br J Surg 1993; 80 : 110-11.
**6.** Thomas et al. The direct perfusion of surgical wounds with local anaesthetic solution : an approach to postoperative pain ? Ann R Coll Surg Engl 1983; 65 : 226-9.
**7.** Levack et al. Abdominal wound perfusion for the relief of postoperative pain. Br J Anaesth 1986; 58 : 615-19.
**8.** Oakley et coll. Randomized placebo-controlled trial of local anaesthetic infusion in day-case inguinal hernia repair. Br J Surg 1998; 85 : 797-9.
**9.** Griffith et coll. Prospective randomized study of a new method of providing postoperative pain relief following femoropopliteal bypass. Br J Surg 1996; 83 : 1735-8.
**10.** Fisher et Meller. Continuous postoperative regional analgesia by nerve sheath block for amputation surgery. A pilot study. Anaest Analg 1991; 72 : 300-3.

## Revendications

1. Structure implantable souple pour la libération prolongée et contrôlée d'un principe actif, constituée d'un support biorésorbable et d'un principe actif intimement lié audit support, et qui présente une cohésion entre le principe actif et le support biorésorbable induite par mouillabilité d'un des composants de la structure, et dans laquelle le support biorésorbable est formé d'un mélange de copolymère amorphe d'acide lactique et d'acide glycolique possédant un rapport massique entre les unités d'acide lactique et d'acide glycolique compris dans la gamme de 80/20 à 20/80, de préférence dans la gamme de 70/30 à 30/70, et de préférence encore égal à 50/50 et de 0,5 à 20 % en masse, de préférence de 5 à 15 % en masse par rapport à la masse du support, d'un plastifiant biocompatible choisi parmi l'acide lactique, un oligomère d'acide lactique, ou un mélange de ces composés, ledit mélange de copolymère et de plastifiant ayant une Tg inférieure ou égale à 15°C.

2. Structure implantable selon la revendication 1, dans laquelle le principe actif est choisi parmi les anesthésiques locaux, les antalgiques morphiniques ou non morphiniques, les facteurs de cicatrisation, les anti-inflammatoires, les antibiotiques, les anti-fongiques, les corticoïdes, les hormones, les antimitotiques, les facteurs de croissance, ou un mélange de ces principes actifs.

3. Structure implantable selon la revendication 2, dans laquelle le principe actif est un anesthésique local.

4. Structure implantable selon l'une des revendications 1 à 3, qui se présente sous la forme de fil, de film, de jonc, de ruban de forme parallélépipédique de base carrée ou rectangle, de mèche, de tissu tissé ou non-tissé, de plaque, de cathéter ou de comprimé voire de tablette ou encore de fil de suture.

5. Structure implantable selon l'une des revendications 1 à 4, qui se présente sous la forme d'une structure sandwich.

6. Procédé de fabrication d'une structure implantable souple pour la libération prolongée et contrôlée d'un principe actif, constituée d'une structure composite homogène avec des interfaces cohérentes, et dans laquelle le support biorésorbable est formé d'un mélange de copolymère amorphe d'acide lactique et d'acide glycolique possédant un rapport massique entre les unités d'acide lactique et d'acide glycolique compris dans la gamme de 80/20 à 20/80, de préférence dans la gamme de 70/30 à 30/70, et de préférence encore égal à 50/50 et de 0,5 à 20 % en masse, de préférence de 5 à 15 % en masse, par rapport à la masse du support, d'un plastifiant biocompatible choisi parmi l'acide lactique, un oligomère d'acide lactique, ou un mélange de ces composés, ledit mélange de copolymère et de plastifiant ayant une Tg inférieure ou égale à 15°C, qui comprend les étapes suivantes :
a) un mélange des produits composant la structure,
b) un passage avec ou sans pression appliquée, dans une chambre de transfert, soit b1) à une température comprise entre la température de fusion du principe actif et la température de transition vitreuse ou de fusion du copolymère, soit b2) à température supérieure à la fois à la température de fusion du principe actif et à la température de transition vitreuse du copolymère, et
c) une mise en forme de la structure implantable sous pression à partir de cet état intermédiaire.

7. Procédé selon la revendication 6, qui comprend en outre d) une étape de traitement thermique.

8. Procédé selon la revendication 6 ou 7, qui est un procédé de moulage par compression-transfert, de moulage par injection transfert, d'extrusion ou de filage avec étape préliminaire de transfert.

9. Procédé selon l'une des revendications 6 à 8, dans lequel le mélange des produits obtenu à l'étape a) est broyé jusqu'à obtenir une granulométrie comprise dans la gamme de 5 à 150 µm, de préférence de 10 à 50 µm.

10. Procédé selon l'une des revendications 6 à 9, dans lequel le principe actif est choisi parmi les anesthésiques locaux, les antalgiques morphiniques ou non morphiniques, les facteurs de cicatrisation, les anti-inflammatoires, les antibiotiques, les anti-fongiques, les corticoïdes, les hormones, les antimitotiques, les facteurs de croissance, ou un mélange de ces principes actifs.

11. Procédé selon la revendication 10, dans lequel le principe actif est un anesthésique local.

## Claims

1. Flexible implantable structure for the sustained and controlled release of an active principle, consisting of a bioabsorbable support and an active principle intimately associated with said support, which exhibits a cohesion between the active principle and the bioabsorbable support that is induced by the wettability of one of the components of the structure, and in which the bioabsorbable support is formed of a mixture of an amorphous lactic acid/glycolic acid copolymer having a weight ratio between the lactic acid and glycolic acid units ranging from about 80/20 to 20/80, preferably ranging from about 70/30 to 30/70 and particularly preferably of 50/50, and about 0.5 to 20% by weight, preferably about 5 to 15% by weight, based on the weight of the support, of a biocompatible plasticizer selected from lactic acid, a lactic acid oligomer and a mixture of these compounds, said mixture of copolymer and plasticizer having a Tg below or equal to 15°C.

2. Implantable structure according to claim 1 in which the active principle is selected from local anesthetics, morphine or non-morphine analgesics, healing factors, anti-inflammatories, antibiotics, antifungals, corticoids, hormones, anti-mitotics, growth factors and a mixture of these active principles.

3. Implantable structure according to claim 2 in which the active principle is a local anesthetic.

4. Implantable structure according to one of claims 1 to 3 which is in the form of a yarn, film, hank, ribbon of parallelepipedal shape with a square or rectangular base, sliver, woven or non-woven fabric, plate, catheter, tablet, sheet or suture thread.

5. Implantable structure according to one of claims 1 to 4 which is in the form of a sandwich structure.

6. Process for the manufacture of a flexible implantable structure for the sustained and controlled release of an active principle, consisting of a homogeneous composite structure with coherent interfaces, in which the bioabsorbable support is formed of a mixture of an amorphous lactic acid/glycolic acid copolymer having a weight ratio between the lactic acid and glycolic acid units ranging from about 80/20 to 20/80, preferably ranging from about 70/30 to 30/70 and particularly preferably of 50/50, and about 0.5 to 20% by weight, preferably about 5 to 15% by weight, based on the weight of the support, of a biocompatible plasticizer selected from lactic acid, a lactic acid oligomer and a mixture of these compounds, said mixture of copolymer and plasticizer having a Tg below or equal to 15°C, said process comprising the following steps:
a) mixing of the component products of the structure,
b) passage, with or without applied pressure, through a transfer chamber, either b1) at a temperature between the melting point of the active principle and the glass transition temperature or melting point of the copolymer, or b2) at a temperature that is above both the melting point of the active principle and the glass transition temperature of the copolymer, and
c) shaping of the implantable structure under pressure from this intermediate state.

7. Process according to claim 6 which also comprises d) a heat treatment step.

8. Process according to claim 6 or 7 which is a process of compression - transfer molding, injection - transfer molding, or extrusion or spinning with a preliminary transfer step.

9. Process according to one of claims 6 to 8 in which the mixture of products obtained in step a) is ground to give a particle size ranging from about 5 to 150 µm, preferably from about 10 to 50 µm.

10. Process according to one of claims 6 to 9 in which the active principle is selected from local anesthetics, morphine or non-morphine analgesics, healing factors, anti-inflammatories, antibiotics, antifungals, corticoids, hormones, anti-mitotics, growth factors and a mixture of these active principles.

11. Process according to claim 10 in which the active principle is a local anesthetic.

## Patentansprüche

1. Implantierbare elastische Struktur zur verzögerten und kontrollierten Freisetzung eines Wirkstoffes, bestehend aus einem bioresorbierbaren Träger und einem Wirkstoff, der innig an den Träger gebunden ist, und welche eine Kohäsion zwischen dem Wirkstoff und dem bioresorbierbaren Träger aufweist, induziert durch Benetzbarkeit von einem der Bestandteile der Struktur, und in welcher der bioresorbierbare Träger aus einem Gemisch von amorphem Copolymer von Milchsäure und Glycolsäure ausgebildet ist, welches ein Masseverhältnis unter den Milchsäureeinheiten und Glycolsäureeinheiten besitzt, das im Bereich von 80/20 bis 20/80, vorzugsweise im Bereich von 70/30 bis 30/70 liegt und weiter bevorzugt gleich 50/50 ist, und 0,5 bis 20 Masseprozent, vorzugsweise 5 bis 15 Masseprozent im Verhältnis zur Masse des Trägers von einem biokompatiblen Weichmachers, gewählt unter Milchsäure, einem Oligomer von Milchsäure oder einem Gemisch dieser Verbindungen, wobei das Gemisch aus Copolymer und Weichmacher eine Tg kleiner oder gleich 15°C hat.

2. Implantierbare Struktur nach Anspruch 1, in welcher der Wirkstoff gewählt ist unter den lokalen Anästhetika, gegebenenfalls morphinischen Schmerzlinderungsmitteln, den Vernarbungsfaktoren, den Antientzündungsmitteln, den Antibiotika, den Fungiziden, den Kortikoiden, den Hormonen, den antimitotischen Mitteln, den Wachstumsfaktoren oder einem Gemisch dieser Wirkstoffe.

3. Implantierbare Struktur nach Anspruch 2, in welcher der Wirkstoff ein lokales Anästhetikum ist.

4. Implantierbare Struktur nach einem der Ansprüche 1 bis 3, die in Form eines Fadens, einer Folie, eines Rohrblattes, eines Bandes mit Parallelepipedform mit Quadrat- oder Rechteckgrundfläche, eines Dochts, eines gegebenenfalls gewebten Gewebes, einer Platte, eines Katheters oder eines Tablette, sogar einer Pastille oder auch eines Nähfadens vorliegt.

5. Implantierbare Struktur nach einem der Ansprüche 1 bis 4, die in Form einer Sandwich-Struktur vorliegt.

6. Verfahren zur Herstellung einer elastischen implantierbaren Struktur zur verzögerten und kontrollierten Freisetzung eines Wirkstoffs, bestehend aus einer homogenen Verbundstruktur mit kohärenten Grenzflächen und in welcher der bioresorbierbare Träger ausgebildet ist aus einem Gemisch von amorphem Copolymer von Milchsäure und Glycolsäure, das ein Masseverhältnis unter den Milchsäure- und Glycolsäureeinheiten besitzt, das im Bereich von 80/20 bis 20/80, vorzugsweise im Bereich von 70/30 bis 30/70 liegt und weiter bevorzugt gleich 50/50 ist, und 0,5 bis 20 Masseprozent, vorzugsweise 5 bis 15 Masseprozent im Verhältnis zur Masse des Trägers von einem biokompatiblen Weichmachers, gewählt unter Milchsäure, einem Oligomer von Milchsäure oder einem Gemisch dieser Verbindungen, wobei das Gemisch von Copolymer und Weichmacher eine Tg unter oder gleich 15°C hat, die die folgenden Eigenschaften umfaßt:
a) ein Mischen der Produkte, die die Struktur bilden,
b) einen Durchgang, gegebenenfalls mit aufgebrachtem Druck, in einer Überführungskammer, entweder b1) bei einer Temperatur zwischen der Schmelztemperatur des Wirkstoffes und der Glasübergangstemperatur oder Schmelztemperatur des Copolymers, oder b2) bei einer Temperatur, die sowohl höher als die Schmelztemperatur des Wirkstoffes als auch die Glasübergangstemperatur des Copolymers ist, und
c) ein Formen der implantierbaren Struktur unter Druck aus diesem Zwischenzustand.

7. Verfahren nach Anspruch 6, das außerdem d) eine Stufe zur thermischen Behandlung umfaßt.

8. Verfahren nach Anspruch 6 oder 7, das ein Verfahren ist zum Formen durch Kompression-Überführung, zum Formen durch Spritzformen, zur Extrusion oder zum Spinnen mit einem vorherigen Überführungsschritt.

9. Verfahren nach einem der Ansprüche 6 bis 8, bei dem das Gemisch der Produkte, das bei Schritt a) erhalten ist, gemahlen wird bis zum Erhalt einer Granulometrie, die im Bereich von 5 bis 150 µm, vorzugsweise 10 bis 50 µm, liegt.

10. Verfahren nach einem der Ansprüche 6 bis 9, bei dem der Wirkstoff gewählt wird unter den lokalen Anästhetika, den gegebenenfalls morphinischen Schmerzlinderungsmitteln, den Vernarbungsfaktoren, den Antientzündungsmitteln, den Antibiotika, den Fungiziden, den Kortikoiden, den Hormonen, den antimitotischen Mitteln, den Wachstumsfaktoren oder einem Gemisch dieser Wirkstoffe.

11. Verfahren nach Anspruch 10, bei dem der Wirkstoff ein lokales Anästhetikum ist.
